# EUROPEAN PATENT SPECIFICATION

(11) **EP 3 596 236 B1**
(45) Date of publication and mention of the grant of the patent: **02.06.2021**
(21) Application number: 18730095.9
(22) Date of filing: 19.03.2018
(51) Int. Cl.: C12Q 1/6886

(54) **MGMT EPIGENETIC DEEP-SEQUENCING ASSAY**
EPIGENETISCHER MGMT-TIEFENSEQUENZTEST
DOSAGE DE SÉQUENÇAGE PROFOND ÉPIGÉNÉTIQUE DE MGMT

(30) Priority: 17.03.2017 US 201762472860 P
(43) Date of publication of application: 22.01.2020
(73) Proprietor: MDxHealth SA, 4040 Herstal (BE)
(72) Inventor: TROOSKENS, Geert, 9000 Gent (BE); VAN CRIEKINGE, Wim, 2550 Waarloos (BE); VANNESTE, Leander, 4040 Herstal (BE); VANDERSMISSEN, Johan, 3730 Hoeselt (BE)
(74) Representative: Boult Wade Tennant LLP
(86) International application number: PCT/IB2018/000385
(87) International publication number: WO 2018/167572

(56) References cited:
- WO-A1-2014/125421
- WO-A2-2016/205233
- CN-B- 102 329 860
- MANABU KANEMOTO ET AL: "Prognostic prediction of glioblastoma by quantitative assessment of the methylation status of the entire MGMT promoter region", BMC CANCER, BIOMED CENTRAL, LONDON, GB, vol. 14, no. 1, 30 August 2014 (2014-08-30), page 641, XP021195718, ISSN: 1471-2407, DOI: 10.1186/1471-2407-14-641

### SEQUENCE LISTING

<110> MDxHealth SA Trooskens, Geert Van Criekinge, Wim Vanneste, Leander Vandersmissen, Johan
<120> MGMT EPIGENETIC DEEP-SEQUENCING ASSAY
<130> 5545-00124
<150> US 62/472,860
   <151> 2017-03-17
<160> 7
<170> PatentIn version 3.5
<210> 1
   <211> 167
   <212> DNA
   <213> Homo sapiens
<400> 1
   ggatatgttg ggatagttcg cgtttttaga acgttttgcg tttcgacgtt cgtaggtttt        60
   cgcggtgcgt atcgtttgcg atttggtgag tgtttgggtc gtttcgtttt cggaagagtg       120
   cggagttttt tttcgggacg gtggtagttt cgagtggttt tgtaggt                     167
<210> 2
   <211> 23
   <212> DNA
   <213> Homo sapiens
<400> 2
   tttcgacgtt cgtaggtttt cgc 23
<210> 3
   <211> 22
   <212> DNA
   <213> Homo sapiens
<400> 3
   ctcgaaacta ccaccgtccc ga 22
<210> 4
   <211> 25
   <212> DNA
   <213> Homo sapiens
<400> 4
   agggagtata taggttgggg aagtt 25
<210> 5
   <211> 27
   <212> DNA
   <213> Homo sapiens
<400> 5
   aacacacaat aacaaacaca aattcac 27
<210> 6
   <211> 18
   <212> DNA
   <213> Homo sapiens
<400> 6
   ggatatgttg ggatagtt 18
<210> 7
   <211> 16
   <212> DNA
   <213> Homo sapiens
<400> 7
   gcctacaaaa ccactc 16

## Description

### BACKGROUND

The field of the invention relates to methods for detecting epigenetic modifications. In particular, the field of the invention relates to methods for detecting methylation in the promoter of the O-6-methylguanine-DNA methyltransferase gene (MGMT) via performing deep-sequencing. The disclosed methods may be performed in order to detect whether a subject exhibits methylation in the promoter MGMT and predict whether the subject will respond to treatment with alkylating agents and/or radiotherapy.

Over 14.000 brain cancer-related deaths, or 2.4% of all cancer-related deaths, are reported per year in the US [1]. Glioblastoma multiforme (GBM) is the highest-grade astrocytoma and the most common and most aggressive form of brain cancer. It can occur de novo or as a secondary glioblastoma in 5 % of the cases [2]. GBM constitutes 30% of all brain tumors and patients have a 5-year survival rate lower than 17% [1].

In normal cells the O-6-methylguanine-DNA methyltransferase gene (MGMT) is responsible for repair of DNA damage caused by ionizing radiation, organic cyclic compounds and oxidative stress through DNA de-alkylation [3]. The MGMT protein removes alkyl groups from the O⁶-position of guanine by an irreversible transfer of the alkyl group to a cysteine residue at its active site. The original guanine nucleotide is thereby restored and the alkylated MGMT protein sent to proteasome-mediated degradation. Thus, the amount of MGMT proteins in a cell correlates directly with the cells ability to repair DNA damage [4, 5, 6, 7]. Epigenetic silencing of MGMT by DNA methylation has been observed in various tumors [8].

When MGMT is silenced, patients showed an increased risk for developing colorectal cancer [9]. Interestingly, epigenetic silencing of MGMT has been associated with longer overall survival in patients with GBM who receive radiotherapy (RT) combined with temozolomide (TMZ) chemotherapy [10, 11]. Approximately 30% to 45% of the patients with gliomas have a methylated MGMT promoter serving as a favorable predictive factor for chemoradiotherapy [12, 13]. The MGMT methylation status can be combined with gene expression and genomic mutations to further enhance the predictive test for GBM patients receiving RT with TMZ [14, 15].

With the increased utilization of next-generation sequencing (NGS), mainly in research settings, the question arises whether this technique offers benefits over PCR-based approaches. Methylation-specific PCR (MSP), a PCR-based technique that can sensitively detect methylated molecules in a background of unmethylated DNA [16], is commonly used to determine DNA methylation. However, MSP typically results in a binary (methylated or unmethylated) or a quantitative overall (qPCR) call of the DNA methylation at the promoter region, lacking single-base methylation quantification. Earlier studies showed great variability in determining the methylation status of the MGMT promoter. Pyrosequencing and Sanger sequencing outperformed other techniques, including MSP as predictor of prognosis in GBM patients [17, 18, 19].

The purpose of this study was to investigate if ultra deep NGS (bisulphite treatment, target amplification, followed by NGS) can be applied as a predictive and/or prognostic method to determine the methylation status of the MGMT promoter. The high coverage attained with NGS has the potential for accurate assessment of the heterogeneity of methylation, both inter- and intra-allele. With the decreasing cost of NGS [20], this technique could be a valuable alternative to MSP at a relatively low cost.

### SUMMARY

Disclosed are methods and systems for detecting methylation of the promoter of O-6-methylguanine-DNA methyltransferase gene (MGMT). In particular, the methods and systems may be utilized to detect methylation in the MGMT promoter in a DNA sample from a glioblastoma to predict whether a subject having the glioblastoma will respond to treatment with an alkylating agent. The methods and systems typically include a step of deep-sequencing the DNA sample after the DNA sample has been treated with a reagent that modifies unmethylated cytosine, wherein detecting methylation of the MGMT promoter comprises detecting methylation of the MGMT promoter at a CpG at position 44 of SEQ ID NO: 1 and/or detecting methylation of the MGMT promoter at a CpG at position 61 of SEQ ID NO: 1.

The present invention further discloses an alkylating agent and/or radiotherapy for use in the treatment of glioblastoma in a subject wherein the subject is selected for treatment on the basis of a method comprising: (a) performing deep-sequencing of the O-6-methylguanine-DNA methyltransferase gene (MGMT) promoter in DNA from a glioblastoma sample of a subject after the DNA has been treated with a reagent that converts an unmethylated cytosine to uracil; and(b) detecting methylation of the MGMT promoter in the DNA, wherein the detected methylation predicts whether the subject will respond to treatment with an alkylating agent and/ or radiotherapy (RT) and wherein detecting methylation of the MGMT promoter comprises detecting methylation of the MGMT promoter at a CpG at position 44 of SEQ ID NO: 1 and/or detecting methylation of the MGMT promoter at a CpG at position 61 of SEQ ID NO: 1.

The disclosed methods typically include detecting methylation of the MGMT promoter by detecting methylation of the MGMT promoter at a CpG at position 44 (chr10, pos 131265519, Human genome version hg19) and/or detecting methylation of the MGMT promoter at a CpG at position 61 44 (chr10, pos 131265536, Human genome version hg19).

The disclosed typically include performing deep-sequencing of the treated DNA sample. In some embodiments of the disclosed methods, performing deep-sequencing comprises completing an average sample coverage of at least about 10³, 10⁴, 10⁵, or 10⁶ reads. Prior to performing deep-sequencing, the DNA sample typically is treated with a reagent that modifies unmethylated cytosines (*e.g.,* a bisulfite reagent). Optionally, the treated DNA may be amplified (*e.g.,* via polymerase chain reaction (PCR) prior to performing deep-sequencing, and optionally amplified using primers that do not hybridize to a sequence comprising a CpG.

### BRIEF DESCRIPTION OF THE FIGURES

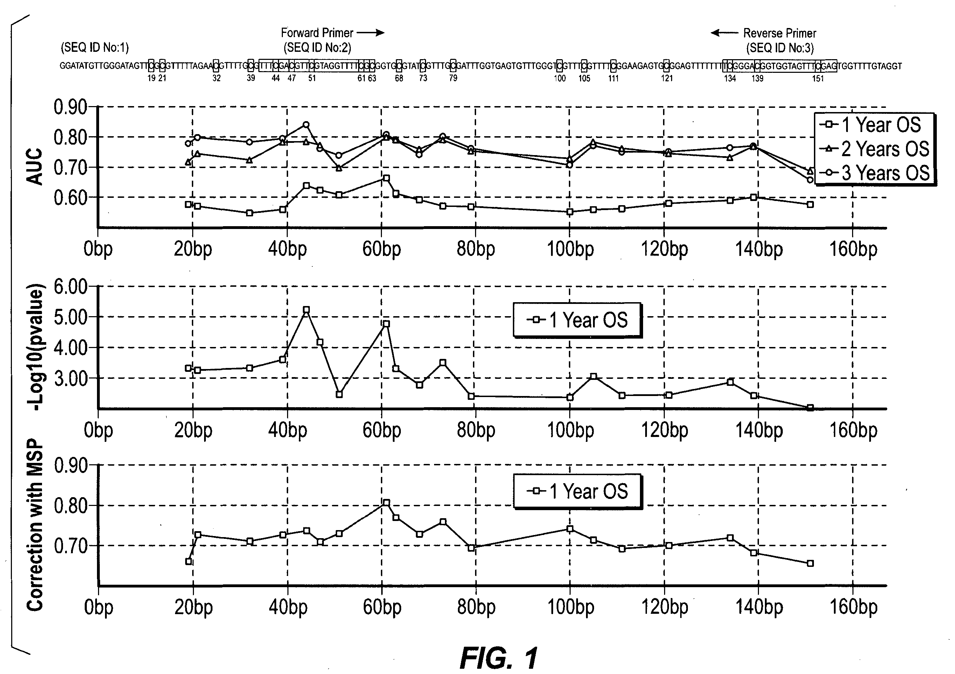

Figure 1 Overview of the BS Sequencing amplicon. The X-axis represents the genomic region sequenced in the MGMT gene promoter region: (A) The Sequencing amplicon (SEQ ID NO:1). The MSP primer pair (SEQ ID NO:2 and SEQ ID NO:3) are labelled and shaded, methylation prone cytosines in a CpG context are shaded and numbered. (B) Survival ROC statistics: The Area under the curve scores for 1, 2, and 3 year overall survival in GBM patients receiving RT + TMZ plotted for each individual CpG. A clear peak can be seen over the three years OS in AUC value for the CpGs at position 44 and 61 (C) The Y-axis represents -log(10) of the p-value for each individual CpG for overall survival in GBM patients receiving RT + TMZ. Higher peaks correspond to lower P-values. A difference in p-values between the CpG methylation ratios of several orders of magnitude was observed. (D) Spearman correlation between the MSP MGMT ratio and the individual CpG methylation levels.

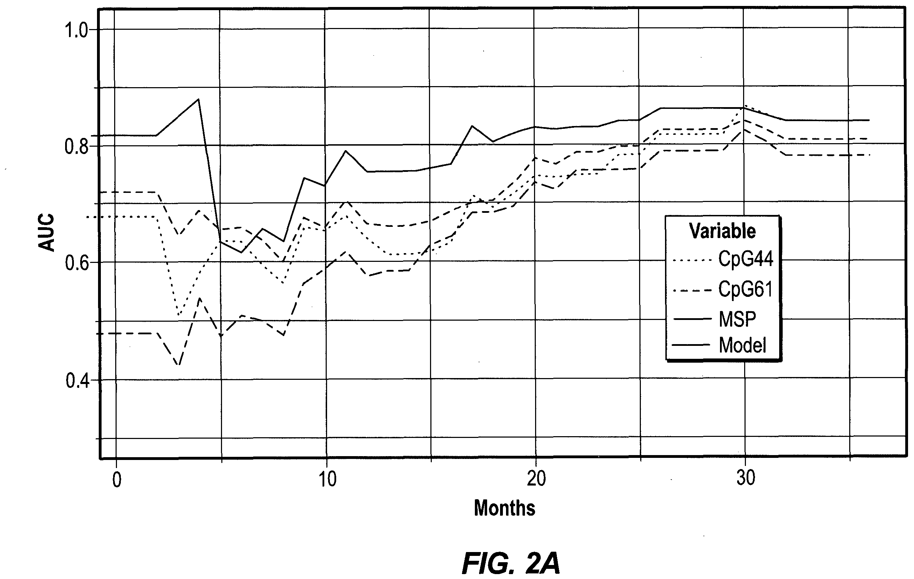

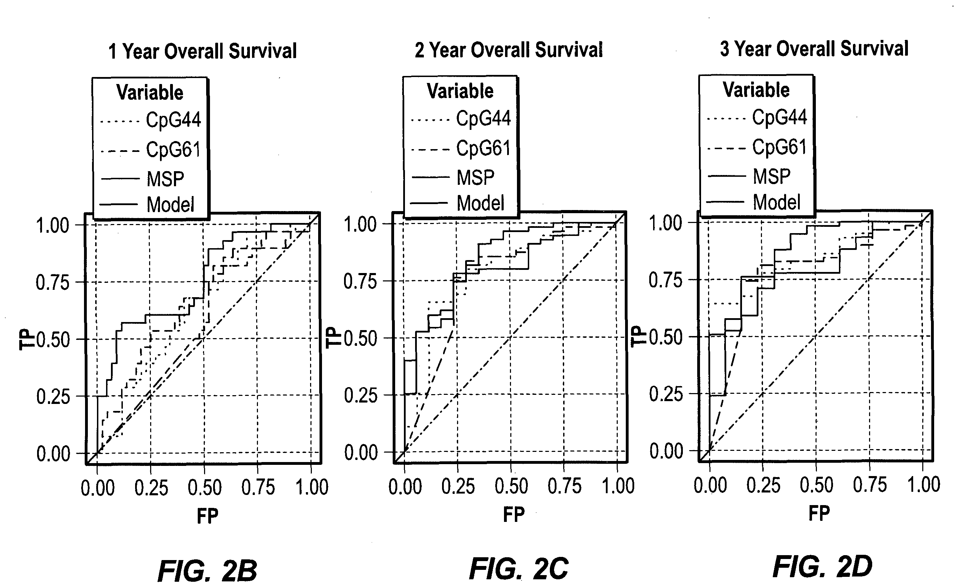

Figure 2 Overview of performance for predicting Overall Survival over time for the MSP assay, the proportional hazard model (Model), CpG44 and CpG61. (A) Area under the ROC curve over time for the four variables. (B) Receiver Operating Characteristic (ROC) curve for predicting overall one year survival, two year survival and three year survival

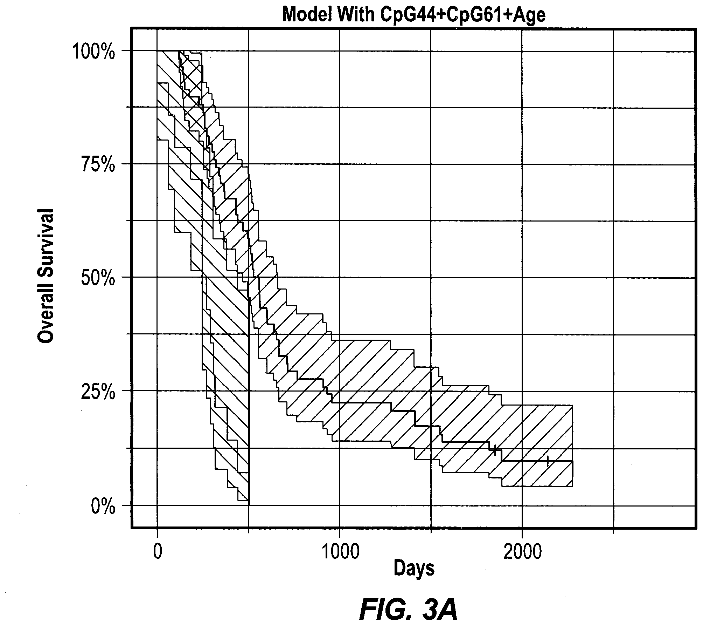

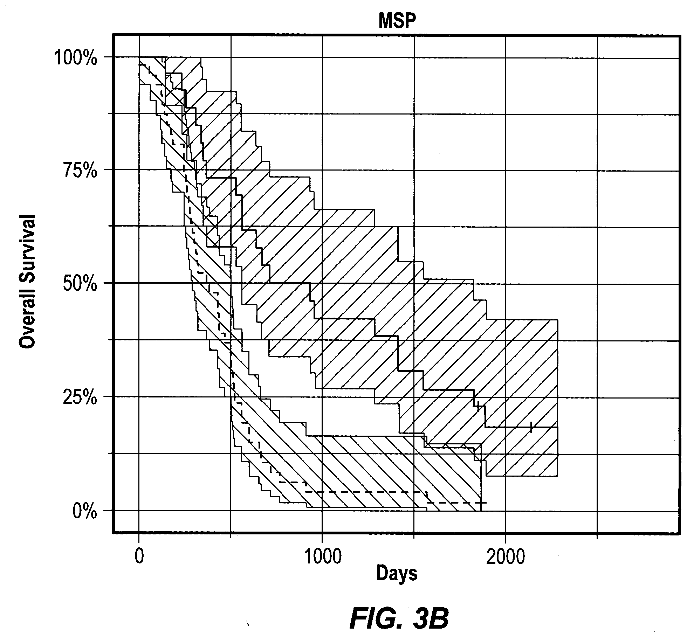

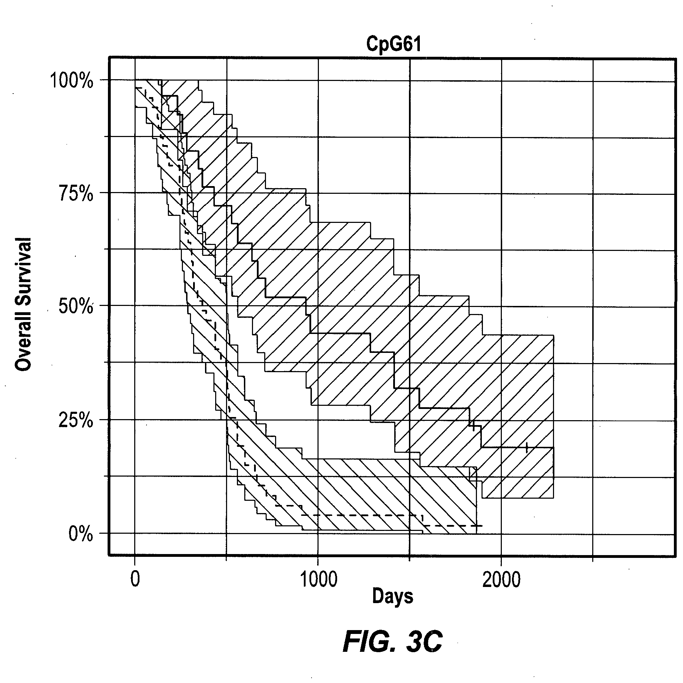

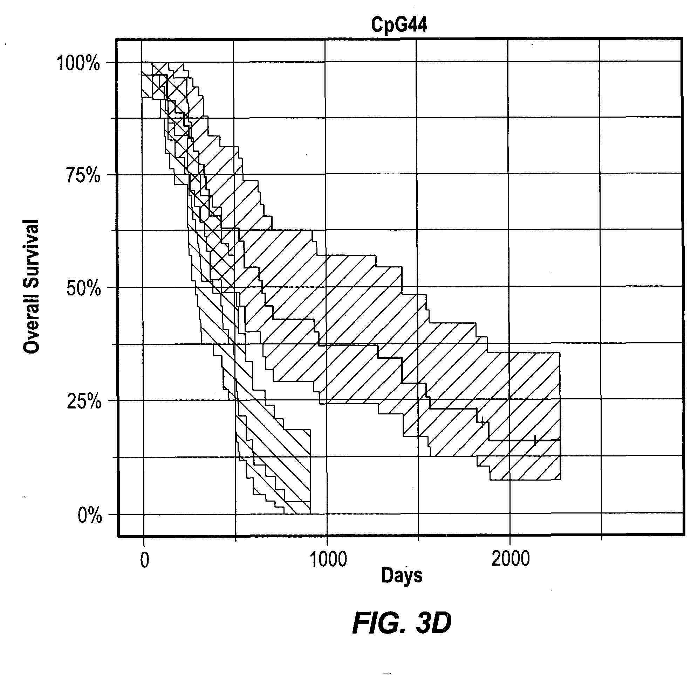

Figure 3 Kaplan-Meier Estimates of Overall Survival (A) The Proportional hazard model (log-rank P=3.45e-07) (B) MGMT Promoter Methylation Status measured by MSP (log-rank P=3.3e-05), (C) The Methylation Status of CpG 61 (P=2.9e-05), (D) The Methylation Status of CpG 44 (P=2.0e-05). The shaded surfaces around the curves indicate the 95% upper and lower confidence interval. P values were calculated using the log-rank test.

### DETAILED DESCRIPTION

The subject matter disclosed herein is described using several definitions, as set forth below and throughout the application.

Unless otherwise specified or indicated by context, the terms "a," "an," and "the," mean "one or more." For example, "a primer," "a CpG site," and "an alkylating agent" should be interpreted to mean "one or more primers," "one or more CpG sites," and "one or more alkylating agents," respectively.

As used herein, "about," "approximately," "substantially," and "significantly" will be understood by persons of ordinary skill in the art and will vary to some extent on the context in which they are used. If there are uses of these terms which are not clear to persons of ordinary skill in the art given the context in which they are used, "about" and "approximately" will mean plus or minus ≤10% of the particular term and "substantially" and "significantly" will mean plus or minus >10% of the particular term.

As used herein, the terms "include" and "including" have the same meaning as the terms "comprise" and "comprising." The terms "comprise" and "comprising" should be interpreted as being "open" transitional terms that permit the inclusion of additional components further to those components recited in the claims. The terms "consist" and "consisting of" should be interpreted as being "closed" transitional terms that do not permit the inclusion of additional components other than the components recited in the claims. The term "consisting essentially of" should be interpreted to be partially closed and allowing the inclusion only of additional components that do not fundamentally alter the nature of the subject matter recited in the claims.

The terms "subject," "patient," and "individual" may be used interchangeably herein.

A "subject in need thereof" may include a subject having or at risk for developing a cell proliferative disease or disorder such as cancer or a tumor. A tumor that involves a tissue or organ of the central nervous system is referred to herein as a "brain tumor." A brain tumor may include a glioma, an anaplastic astrocytoma, a gliobalstoma multiforme, a low grade astrocytoma glioblastoma, a medulloblastoma, an oligodendroglioma or a neuroblastoma, for example. In particular, a subject in need thereof may include a subject having glioblastoma, which may include an aggressive form of astrocytoma such as glioblastoma multiforme (GBM).

The presently disclosed subject matter relates to alkylating agents and the use of alkylating agents for treating diseases and disorders, such as cell proliferative disorders. Alkylating agents are known in the art and include highly reactive molecules that cause cell death by alkylating DNA. The most frequent site of alkylation in DNA is the O⁶ position of guanine which subsequently results in cross-linking between single strands of double-stranded DNA and cell death. The cross-linking of double-stranded DNA by alkylating agents is inhibited by the enzymatic activity of the cellular DNA-repair protein O⁶-methylguanine-DNA methyltransferase (MGMT). The MGMT protein (MGMT (E.C.2.1.1.63), also known as O⁶-alkylguanine-DNA alkyltransferase (AGT)), rapidly reverses the formation of adducts at the O⁶ position of guanine via transfer of the alkyl adduct to a cysteine residue within the MGMT protein, thereby averting the formation of lethal cross-links and other mutagenic effects. Thus, the presence and activity of the enzyme MGMT impedes the activity of alkylating agents in chemotherapy.

The DNA sequence of the *Homo sapiens* MGMT gene is provided at the National Center for Biotechnology Information (NCBI) as Reference Sequence: NG_052673.1. The disclosed methods may include detecting methylation of the sequence of the MGMT gene including its promoter region. In some embodiments, the disclosed methods include detecting methylation of the region of human chromosome 10 between positions 131155505 and 131155619 based on version 36.1 of the National Center for Biotechnology Information (NCBI) human genome. (*See also* sequence provided in Figure 1).

As used herein, the term "alkylating agent" refers to a therapeutic that may be administered to a subject in need thereof, including a subject having glioblastoma, wherein preferably the alkylating agent results in methylation of the DNA of the glioblastoma at an O⁶-guanine position. Alkylating agents may include, but are not limited to, temozolomide (TMZ), carmustine, lomustine, bendamustine, uramustine, cisplatin, carboplatin, mechlorethamine, streptozocin, cyclophosphamide, ifosfamide, melphalan, chlorambucil, busulfan, thiotepa, dacarbazine, procarbazine, altretamine, and mitozolomide.

The disclosed methods may include steps known in the art. For example, the disclosed methods may include steps disclosed in U.S. Patent Nos. 6,773,897 and 7,655,444, and in U.S. Publication No. 2016/0032368.

The disclosed subject matter relates to methods for determining the state of methylation of one or more nucleic acids isolated from a subject. The phrases "nucleic acid" or "nucleic acid sequence" as used herein refer to an oligonucleotide, nucleotide, polynucleotide, or to a fragment of any of these, to DNA or RNA of genomic or synthetic origin which may be single-stranded or double-stranded and may represent a sense or antisense strand. As will be understood by those of skill in the art, when the nucleic acid is RNA, the deoxynucleotides A, G, C, and T are replaced by ribonucleotides A, G, C, and U, respectively.

The nucleic acid can be any nucleic acid where it is desirable to detect the presence of a differentially methylated CpG motifs. The nucleic acid may include, for example, a nucleic acid encoding the enzyme MGMT. The nucleic acid of interest may include the regulatory region of the enzyme gene (e.g., the promoter region) as well as the transcribed regions of the gene. In many genes, CpG motifs are present upstream of a promoter start site and may extend downstream into the transcribed region. Methylation of a CpG motif at a promoter may prevent or inhibit expression of the gene.

In some aspects, the methods contemplated herein may include determining the methylation status of the MGMT gene within a region defined by an upstream boundary of -1000, -900, -800, -700, -600, -500, -400, -300, -200, -100, 0, +100, +200. +300, +400, +500, +600, +700, +800, or +900 nucleotides relative to the transcription start site for the MGMT gene and/or a downstream boundary of -900, -800, -700, -600, -500, - 400, -300, -200, -100, 0, +100, +200. +300, +400, +500, +600, +700, +800, +900, or +1000 nucleotides relative to the transcription start site for the MGMT gene (*e.g.,* a region of -300 - +100). The disclosed methods may include determining the methylation status of DNA that has been treated to convert unmethylated cytosines to uracil (e.g., via bisulfite treatment) and the treated DNA comprises the sequence of SEQ ID NO:1 or a contiguous sequence of SEQ ID NO:1, for example, a sequence comprising at least 10, 20, 30, 40, 50, 60, 70, 80, 90, or 100 contiguous nucleotides of SEQ ID NO:1 (*e.g.,* a region comprising CpG44 and/or CpG61 of SEQ ID NO:1). In some embodiments, the disclosed methods may include determining the methylation status of a region of the DNA of the MGMT promoter to which the primer of SEQ ID NO:2 and/or the primer of SEQ ID NO:3 hybridizes after the DNA has been treated to convert unmethylated cytosines to uracil (e.g., via bisulfite treatment) or a region of the MGMT promoter bounded by and including the primer binding sites of the primer of SEQ ID NO:2 and/or the primer of SEQ ID NO:3 (e.g., a region comprising CpG44 and/or CpG61 of SEQ ID NO:1). In some embodiments, the disclosed methods may include determining the methylation status of a region of the DNA of the MGMT promoter to which the primer of SEQ ID NO:6 and/or the primer of SEQ ID NO:7 hybridizes after the DNA has been treated to convert unmethylated cytosines to uracil (e.g., via bisulfite treatment) or a region of the MGMT promoter bounded by and including the primer binding sites of the primer of SEQ ID NO:6 and/or the primer of SEQ ID NO:7 (e.g., a region comprising CpG44 and/or CpG61 of SEQ ID NO:1 or any CpG detected in SEQ ID NO:1 as listed in Table 1 in the Example section below).

Nucleic acids for use in the disclosed methods may be isolated from a biological sample of a subject as known in the art. In some embodiments, the nucleic acid can be isolated from tumor tissue, brain tissue, cerebrospinal fluid, blood, plasma, serum, lymph, lymph nodes, spleen, liver, bone marrow, or any other biological specimen. Tumor tissue, blood, plasma, serum, lymph, brain tissue, cerebrospinal fluid and bone marrow are obtained by various medical procedures known to those of skill in the art.

The presently disclosed methods may be practiced in order to predict a clinical response, including predicting a clinical response of a tumor (*e.g.,* a glioblastoma) to treatment with a chemotherapeutic agent (*e.g.,* an alkylating agent. Criteria in the art for determining a response to therapy are widely accepted and enable comparisons of the efficacy alternative treatments. A complete response or remission is the disappearance of all detectable malignant disease. A partial response is an approximately 50 percent decrease in the product of the greatest perpendicular diameters of one or more lesions, and there can be no increase in size of any lesion or the appearance of new lesions. Progressive disease means at least an approximately 25 percent increase in the product of the greatest perpendicular diameter of one lesion or the appearance of new lesions. Typically, the response to treatment is evaluated after the subjects had completed therapy.

With respect to response to treatment of gliomas, a complete response may be defined as the absence of any evidence of the tumor on computed tomographic (CT) or magnetic resonance imaging (MRI) scans, for example, with no need for steroid treatment and an improvement in the subject's general condition. Subjects with persistent CT abnormalities but with more than a 50 percent reduction in both the diameter and the volume of the tumor, a reduced need for steroid treatment, and a stabilized neurologic condition are considered to have a partial response. The disease is considered to have progressed if both the diameter and volume of the tumor increased by 25 percent or more of the initial measurements, if a new lesion is evident on CT or MRI scans, or if the subject's neurologic condition worsened and required an increased dose of steroids.

In the disclosed methods, a DNA sample is treated with an agent that modifies unmethylated cytosine. The term "modifies" as used herein includes the conversion of an unmethylated cytosine to another nucleotide which will facilitate methods to distinguish the unmethylated from the methylated cytosine. Preferably, the agent does not modify methylated cytosine. Preferably, the agent modifies unmethylated cytosine to uracil. Preferably, the agent used for modifying unmethylated cytosine is sodium bisulfite. However, other agents that similarly modify unmethylated cytosine, but not methylated cytosine can also be used in the method. Sodium bisulfite (NaHSO₃) reacts readily with the 5,6-double bond of cytosine, but poorly with methylated cytosine. Cytosine reacts with the bisulfite ion to form a sulfonated cytosine reaction intermediate that is susceptible to deamination, giving rise to a sulfonated uracil. The sulfonate group can be removed under alkaline conditions, resulting in the formation of uracil. Uracil is recognized as a thymine by Taq polymerase and therefore upon PCR, the resultant product contains cytosine only at the position where 5-methylcytosine occurs in the starting template DNA.

The above disclosure generally describes the present invention. A more complete understanding can be obtained by reference to the following specific examples provided herein for purposes of illustration only and are not intended to limit the scope of the invention.

### EXAMPLES

### Title - MGMT Epigenetic Sequencing Assay Predicts Overall Survival in Glioblastoma Patients Receiving Temozolomide

Reference is made to the manuscript entitled, "MGMT Epigenetic Sequencing Assay Predicts Overall Survival in Glioblastoma Patients Receiving Temozolomide," Geert Trooskens, Annika Malmström, Martin Hallbeck, Peter Söderkvist, Greg Jones, Johan Vandersmissen, Hendrik Van De Voorde, Leander Van Neste and Wim Van Criekinge, submitted for publication.

### Abstract

Background: Epigenetic silencing of MGMT is associated with longer overall survival in patients with Glioblastoma multiforme who receive radiotherapy (RT) combined with temozolomide (TMZ) chemotherapy. Methylation-specific PCR (MSP) is the current standard used to determine the epigenetic status of the MGMT promoter. The purpose of this study was to investigate if next-generation sequencing (NGS) of targeted bisulphite treated DNA can be used as an alternative method to the MSP assay, allowing assessment of the heterogeneity of methylation in the MGMT promoter.

Methods: A total of 112 Glioblastoma samples from the Linköping University (Sweden) were analyzed using Methylation Specific PCR and Next Generation Sequencing of Amplified Bisulphite treated DNA of the same region. 72 received Radiotherapy combined with temozolomide (TMZ) treatment. MSP ratio cutoffs and individual CpG methylation cutoffs were optimized based on overall survival log-rank P-value. In addition, a hazard model was generated with the individual CpG methylation values and the age variable. Cutoff independent Receiver Operator Characteristic (ROC) analysis was performed on multiple time points.

Results: Ultra deep NGS sequencing showed similar performance predicting overall survival in GBM patients receiving TMZ + RT compared to MSP for both the cutoff dependent analysis by Kaplan-Meier (log-rank test and hazard ratio) (CpG44: P=2.03e-05, HR 0.31; CpG61: P=2.9e-05, HR 0.31; MSP: P=3.30e-05, HR 0.32) and the cutoff independent analysis by area under the receiver operating curve for 1 year, 2 year and 3 year survival respectively (CpG44 AUCs: 0.64, 0.78, 0.84; CpG61 AUCs: 0.66, 0.80, 0.81; MSP AUCs: 0.57, 0.76, 0.78). The multivariate model comprising methylation frequency of two CpG dinucleotides and the patients age variable outperformed the individual methylation frequencies measured by NGS and the MSP assay (Model: P=3.4e-07, HR 0.20, AUCs: 0.75, 0.84, 0.84). Correlation of the methylation frequencies of the individual CpGs with the MSP assay revealed that the forward primer is the main driver of the assay.

Conclusions: NGS is a promising alternative to MSP by matching, and when used in a model combined with the patient's age, exceeding the performance of MSP to predict overall survival by measuring MGMT promoter methylation on a single-nucleotide level.

Keywords: MGMT; Glioblastoma; DNA methylation; Radiotherapy (RT); Temozolomide (TMZ); Next Generation Sequencing (NGS); Survival.

### Methods

Samples. A total of 112 GBM samples from the Linköping University Hospital (Sweden) were analyzed using MSP and NGS. The majority of the samples were collected between 2008 and 2012, however, 10 were older, dating back to 2003. All cases were reassessed to confirm the diagnosis. 72 patients received RT combined with TMZ treatment.

Sample Preparation. A total of four ten-micron formalin-fixed paraffin-embedded (FFPE) slides were obtained from all patients. The tumor areas were separated from benign tissue before DNA isolation using the phenol-chloroform method. The DNA was bisulfite treated using the EZ DNA Methylation kit (Zymo Research).

Direct, Real-Time MSP. MGMT and ACTB quantification was performed by real-time MSP assays. These consisted of parallel amplification/quantification processes using specific primer and primer/detector pairs for each analyte using the Amplifluor assay format on an ABI Prism 7900HT instrument (Applied Biosystems, Foster City, CA). The Amplifluor direct forward primers are preceded by the detection elements (underlined). Sequence details for both forward and re- verse primers are as follows: forward primer MGMT: 5'-TTCGACGTTCGTAGGTTTTCGC-3' (SEQ ID NO:2); reverse primer MGMT: '5-CTCGAAACTACCACCGTCCCGA-3' (SEQ ID NO:3); forward primer ACTB: 5'-AGGGAGTATATAGGTTGGGGAAGTT-3' (SEQ ID NO:4); reverse primer ACTB: 5'-AACACACAATAACAAACACAAATTCAC-3' (SEQ ID NO:5). The MGMT target sequence is located on the sense strand of chromosome 10 between positions 131265515 and 131265629. ACTB target sequence resides on the anti-sense strand of chromosome 7 between positions 5571902 and 5571799, based on version 37.2 of the NCBI human genome. MSP reactions were performed using 1.5 µg of input DNA as described previously [21] and ratios were calculated using the ACTB control gene.

Target Amplification and Sequencing. 50 ng of all samples was used for target amplification. Flanking primes, without CpG's were designed that span the entire region of the MSP assay (5'- GGATATGTTGGGATAGTT-3' (SEQ ID NO:6, 5'-GCCTACAAAACCACTC-3' (SEQ ID NO:7), Integrated DNA Technologies, Leuven, Belgium) covering 19 CpG's. Each bisulfite deep-sequencing amplicon was generated using the FastStart High Fidelity PCR System (Roche) in a 50 µl reaction and a touchdown PCR at annealing temperatures from 60°C and 55°C (five cycles at each temperature) followed by 30 cycles at an annealing temperature of 52°C. Reactions were performed in the GENEAMP PCR system 9700 (Applied Biosystems). After amplification each amplicon was qualified for the expected length using capilair electrophoresis with the Caliper Labchip GX (HT1000 DNA chip). Amplicons were quantified with a PicoGreen assay (Quant-iT PicoGreen dsDNA Assay Kit, Invitrogen-Molecular Probes, P7589) after column purification (High Pure PCR Cleanup Micro Kit, Roche). Next, a pooled and indexed library was prepared using the TruSeq DNA Sample Prep Kit (Illumina), starting with 200 ng of DNA for the end repair reaction. After library preparation, all samples containing adaptor and index sequences were quantified and an equimolar pool was generated. Samples and the artificial amplicon were sequenced on the MiSeq in 5 runs using 24 different indexes. The MiSeq v2 Reagent Kit (Illumina), was used for paired-end sequencing with two times 251 cycles.

Mapping and Analysis. The paired end 251bp sequence reads were trimmed and aligned using the Smith-Waterman algorithm. No mismatches were allowed (in the non-CpGs) . We used the human genome build GRCh37/hg19 as a reference. A methylation percentage was obtained for every CpG within the target amplicon for all samples.

Statistical analysis. MSP ratio cutoffs and CpG methylation cutoffs were optimized based on overall survival log-rank p-value. A Cox proportional-hazards model was generated with the methylation values of the individual CpG's and the patients age variable. Overall survival curves for the MSP ratio, each CpG methylation variable and the model were estimated by the Kaplan-Meier (KM) technique and compared with use of the two-sided log-rank test [22]. An additional methylation cutoff-independent analysis was performed: Time-dependent receiver operating characteristic (ROC) curves and corresponding area under the ROC curve values were calculated from censored survival data using the KM method [23]. Statistical analyses were performed with R, a free software environment available at http://www.r-project.org/.

### Results

MSP. An average of 2986 ng of DNA (median: 2114 ng) was extracted from the total of 112 GBM samples. 72 of the GBM specimen received TMZ+RT and were used in the subsequent survival analysis. With a limit of detection of 10 MGMT copies, all unmethylated samples were clearly lacking amplifiable alleles, with an average of less than 1 MGMT allele per sample. An optimal cutoff for the normalized methylation ratio (amount of mgmt copies/amount of β-actin copies*1000) of 0.61 showed the highest difference in survival (p=3.30E-05 by the log-rank test, Figure 3 B), corresponding to 36% methylated samples.

Ultra Deep Sequencing. An average coverage of 659,000 reads was obtained for the GBM samples. On average, 29% of the reads could be unambiguously mapped and passed the quality filter. The low mapping percentage is due to the strict quality filter applied (no mismatches over the entire amplicon) and a considerable amount of the synthetic control gene spiked in each sample (16.5% of the reads). Two samples showed a significantly (<10000) lower amount of mapped reads (Sample 24020; 2765 reads, sample 24174; 640 reads). Both of them did not receive the RT + TMZ treatment and were excluded in the downstream analysis.

Correlation between NGS and MSP Results. The Spearman correlation between the MSP ratio and the observed methylation frequency in the NGS data of each individual CpG in the 110 samples was calculated and plotted on the genomic coordinates (Figure 1 D). The highest correlation was found for the two most 3' CpGs in the forward primer. Interestingly, the reverse primer showed lower correlation with the MSP assay, with the highest correlated CpG located at the most 3'-end of the reverse primer, while the methylation frequency of the remaining two CpG's seemed to have less influence. In summary, this indicates that the methylation frequency of only a few CpG's are influential in driving the MSP result.

Survival analysis using proportional regression model of NGS methylation data. A Cox proportional Hazard model containing the highest scoring CpG sites by the log-rank test (CpG44 and CpG61), together with the patients age variable was generated to predict the overall survival in the cohort. The performance parameters of the model were consistently higher then the individual CpG sites in the NGS data and the MSP assay in both the cutoff-dependent analysis (Table 1) and cutoff independent analysis at the different time-points (Figure 3 A).

**Table 1. Overview of the Hazard Ratios with 95% confidence intervals and the log-rank p-value of the individual CpG's, the MSP assay and the proportional hazard model.**

| Variable | P-value | HR | 95 CI Low | 95 CI High |
|---|---|---|---|---|
| CpG19 | 5.47E-04 | 0.41 | 0.25 | 0.69 |
| CpG21 | 6.27E-04 | 0.42 | 0.25 | 0.70 |
| CpG32 | 5.03E-04 | 0.42 | 0.25 | 0.69 |
| CpG39 | 3.04E-04 | 0.40 | 0.24 | 0.67 |
| CpG44 | 2.03E-05 | 0.31 | 0.17 | 0.54 |
| CpG47 | 8.96E-05 | 0.37 | 0.22 | 0.62 |
| CpG51 | 3.37E-03 | 0.50 | 0.30 | 0.82 |
| CpG61 | 2.95E-05 | 0.31 | 0.18 | 0.55 |
| CpG63 | 7.12E-04 | 0.41 | 0.24 | 0.70 |
| CpG68 | 5.92E-03 | 0.49 | 0.29 | 0.82 |
| CpG73 | 1.34E-03 | 0.37 | 0.22 | 0.62 |
| CpG79 | 3.88E-03 | 0.48 | 0.28 | 0.80 |
| CpG100 | 6.84E-03 | 0.51 | 0.31 | 0.84 |
| CpG105 | 1.90E-03 | 0.46 | 0.28 | 0.76 |
| CpG111 | 3.74E-03 | 0.49 | 0.30 | 0.80 |
| CpG121 | 3.75E-03 | 0.46 | 0.27 | 0.79 |
| CpG134 | 3.24E-03 | 0.44 | 0.26 | 0.77 |
| CpG139 | 1.45E-03 | 0.44 | 0.26 | 0.74 |
| CpG151 | 3.32E-03 | 0.59 | 0.36 | 0.96 |
| MSP | 5.03E-05 | 0.32 | 0.18 | 0.56 |
| Model | 5.03E-07 | 0.20 | 0.10 | 0.40 |

Comparing Next Generation Sequencing and MSP as a Predictive Marker for Overall Survival. By using ultra deep bisulphite sequencing we identified two CpGs with a similar Hazard Ratio for survival in the 72 GBM patients receiving RT and TMZ (CPG44 with P=2.03e-05 by the log-rank test, hazard ratio 0.31; 95% confidence interval, 0.17 to 0.54 and CpG61 with P=2.9e-05 by the log-rank test, hazard ratio 0.31; 95% confidence interval, 0.18 to 0.55) compared to MSP (P=3.30e-05 by the log-rank test, hazard ratio 0.32; 95% confidence interval, 0.18 to 0.56) (Table 1). The proportional hazard model containing the CpG44, CpG61 and age variable outperformed the MSP assay and the single CpG methylation values (P=3.4e-07 by the log-rank test, hazard ratio 0.20; 95% confidence interval, 0.10 to 0.40). The cutoff-independent analysis generated similar results. The Receiver operating characteristic (ROC) curve analysis for one year, two year and three year OS generated the following Area's under the curve (AUC):
1 Year OS AUCs: 0.57 MSP, 0.64 CpG44, 0.66 CpG61, 0.75 Model;
2 Year OS AUCs: 0.76 MSP, 0.78 CpG44, 0.80 CpG61, 0.84 Model; and
3 Year OS AUCs: 0.78 MSP, 0.84 CpG44, 0.81 CpG61, 0.84 Model.

Next to individual CpG analysis, we looked for DNA methylation patterns on the same allele (intra-allele) that were predictive for overall survival. None were found that performed equally or better to the individual CpGs.

### Discussion

While MSP is a the current golden standard to detect DNA methylation of MGMT in GBM tumor specimen [21], our data suggests that ultra deep NGS sequencing has similar performance compared to MSP. It generates single base, quantitative measurements in GBM samples receiving RT in combination with TMZ chemoradiotherapy.

A challenge when comparing different techniques to measure methylation levels lies in defining a threshold to classify a sample as methylated/unmethylated. The Gel Electrophoresis-Based MSP assay [12] has an inherent binary cutoff by visualizing the actual PCR product. The real-time PCR assay [21] provides us with a continuous methylation ratio variable. The ultra-deep sequencing assay generates a quantitative methylation fraction for every sequenced position in the region of interest.

We chose the optimal thresholds for both the MSP ratio and the individual CpG methylation levels based on the log-rank test. A disadvantage is the direct influence of the cutoff on the proportional amount of patients in each group. The fraction of patients that will be classified as positive is not only a statistical question, but is part of a larger medical decision-making process based on different factors such as overall quality of life, cost and the availability of alternative treatments [24]. Therefore we also compared the MSP assay to NGS with threshold independent measurements (ROC analysis) after defined periods.

The comparison between the MSP assay and the NGS results revealed that only two out of the 19 individual CpGs, both located within the forward primer, showed marginally higher performance. This indicates that the MSP assay [21, 12] is already highly optimized to detect the relevant methylation in the promoter region.

A proportional hazard model incorporating the deep sequencing measurements with the patients 'age at diagnosis' demonstrated consistently higher performance compared to the individual measurements, outperforming the MSP assay and the individual CpG measurements (Table 1, Figure). Adding clinical variables to a biomarker test has been shown to notably improve the precision of the test [25]. However, caution should be exercised for over-fitting when generating a multivariate model from a relatively small dataset (72 samples).

Correlation with MSP suggests that methylation status in the primers 3'-end drives the MSP assay, with the 3'-end CpGs of the forward primer being the most influential ones. Combining the MSP correlation results with the performance of the individual CpGs, one can conclude that the forward primer is located in the region with the highest performance in predicting OS. Indeed, when these CpGs are methylated, a high MSP ratio will be obtained. However, when they have a low methylation frequency, it will affect primer binding resulting in a lower MSP ratio.

A limitation of this study was the relatively small amount of patients in this cohort that received combinational chemoradiotherapy with TMZ (72 samples). In order to use sequencing as an alternative for MSP, the coverage depth needs to be adequate to assure that the alleles with low methylation frequencies can be observed when they are present in the sample while still remaining economically feasible.

### Conclusions

We have shown that ultra-deep bisulfite sequencing can be considered as a valuable alternative, providing quantitative measurements of individual CpG methylation levels, compared to methylation specific PCR. We found two CpGs that matches the performance of the current MSP test in predicting overall survival for GBM patients receiving combinational RT with TMZ treatment. A model that combines methylation levels from NGS data with the patients age shows potential to increase the performance of the test further. As next-generation sequencing becomes a routine part of health care, it can turn into an economically feasible, accurate and reliable technique to detect the MGMT promoter methylation status in glioblastoma multiforme patients.

### References

1. Siegel, R., Ma, J.M., Zou, Z.H., and Jemal A., Cancer Statistics, 2014. CA Cancer J Clin. 2014 Jan-Feb;64(1):9-29.
2. Ohgaki, H., Dessen, P., Jourde, B., Horstmann, S., Nishikawa, T., Di Patre, P.-L., Burkhard, C., Sch¨uler, D., Probst-Hensch, N.M., Maiorka, P.C., Baeza, N., Pisani, P., Yonekawa, Y., Yasargil, M.G., L¨utolf, U.M., Kleihues, P.: Genetic pathways to glioblastoma: a population-based study. Cancer Research 64(19), 6892-6899 (2004).
3. Wood, R.D., Mitchell, M., Sgouros, J., Lindahl, T.: Human DNA Repair Genes. Science (New York, N.Y.) (2001).
4. Rivera, A.L., Pelloski, C.E., Gilbert, M.R., Colman, H., De La Cruz, C., Sulman, E.P., Bekele, B.N., Aldape, K.D.: MGMT promoter methylation is predictive of response to radiotherapy and prognostic in the absence of adjuvant alkylating chemotherapy for glioblastoma. Neuro-Oncology 12(2), 020-121 (2009).
5. Esteller, M., Herman, J.G.: Generating mutations but providing chemosensitivity: the role of O6-methylguanine DNA methyltransferase in human cancer. Oncogene. 2004 Jan 8;23(1):1-8.
6. Gerson, S.L.: MGMT: its role in cancer aetiology and cancer therapeutics. Nature reviews. Cancer 4(4), 296-307 (2004).
7. Wick, W., Weller, M., van den Bent, M., Sanson, M., Weiler, M., von Deimling, A., Plass, C., Hegi, M., Platten, M., Reifenberger, G.: MGMT testing-the challenges for biomarker-based glioma treatment. Nature Reviews. Neurology 10(7), 372-385 (2014).
8. Esteller, M., Sanchez-Cespedes, M., Rosell, R., Sidransky, D., Baylin, S.B., Herman, J.G.: Detection of Aberrant Promoter Hypermethylation of Tumor Suppressor Genes in Serum DNA from Non-Small Cell Lung Cancer Patients. Cancer Research 59(1), 67-70 (1999).
9. Farzanehfar, M., Vossoughinia, H., Jabini, R., Tavassoli, A., Saadatnia, H., Khorashad, A.K., Ahadi, M., Afzalaghaee, M., Karimiani, E.G., Mirzaei, F., Ayatollahi, H.: Evaluation of Methylation of MGMT (O6-Methylguanine-DNA Methyltransferase) Gene Promoter in Sporadic Colorectal Cancer. DNA Cell Biol. 32 (7), 371-377 (2013).
10. Esteller, M., Garcia-Foncillas, J., Andion, E., Goodman, S.N., Hidalgo, O.F., Vanaclocha, V., Baylin, S.B., Herman, J.G.: Inactivation of the DNA-Repair Gene MGMT and the Clinical Response of Gliomas to Alkylating Agents. N Engl J Med. 2000 Nov 9;343(19): 1350-4.
11. Hegi, M.E., Diserens, A.-C., Godard, S., Dietrich, P.-Y., Regli, L., Ostermann, S., Otten, P., Van Melle, G., de Tribolet, N., Stupp, R.: Clinical Trial Substantiates the Predictive Value of O-6-Methylguanine-DNA Methyltransferase Promoter Methylation in Glioblastoma Patients Treated with Temozolomide. Clin Cancer Res. 2004 Mar 15; 10(6): 1871-4.
12. Hegi, M.E., Diserens, A.-C., Gorlia, T., Hamou, M.-F., de Tribolet, N., Weller, M., Kros, J.M., Hainfellner, J.A., Mason, W., Mariani, L., Bromberg, J.E.C., Hau, P., Mirimanoff, R.O., Cairncross, J.G., Janzer, R.C., Stupp, R.: MGMT gene silencing and benefit from temozolomide in glioblastoma. N Engl J Med. 352(10), 997-1003 (2005).
13. Stupp, R., Hegi, M.E., Mason, W.P., van den Bent, M.J., Taphoorn, M.J., Janzer, R.C., Ludwin, S.K., Allgeier, A., Fisher, B., Belanger, K., Hau, P., Brandes, A.A., Gijtenbeek, J., Marosi, C., Vecht, C.J., Mokhtari, K., Wesseling, P., Villa, S., Eisenhauer, E., Gorlia, T., Weller, M., Lacombe, D., Cairncross, J.G., Mirimanoff, R.-O.: Effects of radiotherapy with concomitant and adjuvant temozolomide versus radiotherapy alone on survival in glioblastoma in a randomized phase III study: 5-year analysis of the EORTC-NCIC trial. Lancet Oncol. 10(5), 459-466 (2009).
14. Tini, P., Pastina, P., Nardone, V., Sebaste, L., Toscano, M., Miracco, C., Cerase, A., Pirtoli, L.: The combined EGFR protein expression analysis refines the prognostic value of the MGMT promoter methylation status in glioblastoma. Clin Neurol Neurosurg. 149, 15-21 (2016)
15. Brennan, C.W., Verhaak, R.G.W., McKenna, A., Campos, B., Noushmehr, H., Salama, S.R., Zheng, S., Chakravarty, D., Sanborn, J.Z., Berman, S.H., Beroukhim, R., Bernard, B., Wu, C.-J., Genovese, G., Shmulevich, I., Barnholtz-Sloan, J., Zou, L., Vegesna, R., Shukla, S.A., Ciriello, G., Yung, W.K., Zhang, W., Sougnez, C., Mikkelsen, T., Aldape, K., Bigner, D.D., Van Meir, E.G., Prados, M., Sloan, A., Black, K.L., mEschbacher, J., Finocchiaro, G., Friedman, W., Andrews, D.W., Guha, A., Iacocca, M., O'Neill, B.P., Foltz, G., Myers, J., Weisenberger, D.J., Penny, R., Kucherlapati, R., Perou, C.M., Hayes, D.N., Gibbs, R., Marra, M., Mills, G.B., Lander, E., Spellman, P., Wilson, R., Sander, C., Weinstein, J., Meyerson, M., Gabriel, S., Laird, P.W., Haussler, D., Getz, G., Chin, L.: The Somatic Genomic Landscape of Glioblastoma. Cell 155(2), 462-477 (2013)
16. Herman J.G., Graff, J.R., Myöhännen, S., Nelkin, B.D., and Baylin, S.B. : Methylation-specific PCR: a Novel PCR Assay for Methylation Status of CpG Islands (1996)
17. Quillien, V., Lavenu, A., Karayan-Tapon, L., Carpentier, C., Labussi'ere, M., Lesimple, T., Chinot, O., Wager, M., Honnorat, J., Saikali, S., Fina, F., Sanson, M., Figarella-Branger, D.: Comparative assessment of 5 methods (methylation-specific polymerase chain reaction, methylight, pyrosequencing, methylation-sensitive high-resolution melting, and immunohistochemistry) to analyze O6-methylguanine-DNA-methyltranferase in a series of 100 glioblastoma patients. Cancer 118(17), 4201-4211 (2012).
18. Håvik, A.B., Brandal, P., Honne, H., Dahlback, H.-S.S., Scheie, D., Hektoen, M., Meling, T.R., Helseth, E., Heim, S., Lothe, R.A., Lind, G.E.: MGMT promoter methylation in gliomas-assessment by pyrosequencing and quantitative methylation-specific PCR. J Transl Med. 10(1), 36 (2012).
19. Kanemoto, M., Shirahata, M., Nakauma, A., Nakanishi, K., Taniguchi, K., Kukita, Y., Arakawa, Y., Miyamoto, S., Kato, K.: Prognostic prediction of glioblastoma by quantitative assessment of the methylation status of the entire MGMT promoter region. BMC Cancer 14(1), 641 (2014).
20. Muir, P., Li, S., Lou, S., Wang, D., Spakowicz, D.J., Salichos, L., Zhang, J., Weinstock, G.M., Isaacs, F., Rozowsky, J., Gerstein, M.: The real cost of sequencing: scaling computation to keep pace with data generation. Genome Biology 17(1), 4731 (2016).
21. Vlassenbroeck, I., Califice, S., Diserens, A.-C., Migliavacca, E., Straub, J., Di Stefano, I., Moreau, F., Hamou, M.-F., Renard, I., Delorenzi, M., Flamion, B., DiGuiseppi, J., Bierau, K., Hegi, M.E.: Validation of real-time methylation-specific PCR to determine O6-methylguanine-DNA methyltransferase gene promoter methylation in glioma. The Journal of molecular diagnostics : J Mol Diagn. 10(4), 332-337 (2008).
22. Heller, G.Z.: Survival Analysis: Techniques For Truncated And Censored Data, 2nd Edition. John P. Klein and Melvin L. Moeschberger, Springer-Verlag, New York, 2003. Hardcover. No. of pages: xv +536. ISBN 0-387-95399-X. Statistics in Medicine 23(6), 1020-1021 (2004)
23. Heagerty, P.J., Zheng, Y.: Survival Model Predictive Accuracy and ROC Curves. Biometrics 61(1), 92-105 (2005).
24. Subtil, F., Rabilloud, M.: Estimating the optimal threshold for a diagnostic biomarker in case of complex biomarker distributions. BMC Medical Informatics and Decision Making 14(1), 53 (2014).
25. Van Neste, L., Hendriks, R.J., Dijkstra, S., Trooskens, G., Cornel, E.B., Jannink, S.A., de Jong, H., Hessels, D., Smit, F.P., Melchers, W.J.G., Leyten, G.H.J.M., de Reijke, T.M., Vergunst, H., Kil, P., Knipscheer, B.C., Hulsbergen-van de Kaa, C.A., Mulders, P.F.A., van Oort, I.M., Van Criekinge, W., Schalken, J.A.: Detection of High-grade Prostate Cancer Using a Urinary Molecular Biomarker-Based Risk Score. Eur Urol. 70(5), 740-748 (2016)

## Claims

1. A method comprising:
(a) performing deep-sequencing of the O-6-methylguanine-DNA methyltransferase gene (MGMT) promoter in DNA from a glioblastoma sample of a subject after the DNA has been treated with a reagent that converts an unmethylated cytosine to uracil; and
(b) detecting methylation of the MGMT promoter in the DNA,
wherein the detected methylation predicts
whether the subject will respond to treatment with an alkylating agent and/or radiotherapy (RT) and
wherein detecting methylation of the MGMT promoter comprises detecting methylation of the MGMT promoter at a CpG at position 44 of SEQ ID NO: 1 and/or detecting methylation of the MGMT promoter at a CpG at position 61 of SEQ ID NO:1.

2. The method of claim 1, wherein the alkylating agent is selected from the group consisting of temozolomide, carmustine, lomustine, bendamustine, uramustine, cisplatin, carboplatin, mechlorethamine, streptozocin, cyclophosphamide, ifosfamide, melphalan, chlorambucil, busulfan, thiotepa, dacarbazine, procarbazine, altretamine, and mitozolomide.

3. The method of claim 1, wherein the alkylating agent is temozolomide.

4. An alkylating agent and/or radiotherapy for use in the treatment of glioblastoma in a subject wherein the subject is selected for treatment on the basis of a method as claimed in any one of claims 1 to 3.

5. The alkylating agent and/or radiotherapy for use of claim 4,
wherein the alkylating agent is selected from the group consisting of temozolomide, carmustine, lomustine, bendamustine, uramustine, cisplatin, carboplatin, mechlorethamine, streptozocin, cyclophosphamide, ifosfamide, melphalan, chlorambucil, busulfan, thiotepa, dacarbazine, procarbazine, altretamine, and mitozolomide.

6. The alkylating agent and/or radiotherapy for use of claim 4, wherein the alkylating agent is temozolomide.

7. The method of any of claims 1 to 3, wherein prior to performing deep-sequencing, the treated DNA is amplified.

8. The method of any of claims 1 to 3, wherein prior to performing deep-sequencing, the treated DNA is amplified using primers that do not distinguish between methylated and non-methylated DNA.

9. The method of any of claims 1 to 3, wherein performing deep-sequencing comprises completing an average sample coverage of at least about 10³ reads.

10. Use of a system or kit for performing the method of any of claims 1 to 3, the system or kit comprising one or more components for performing deep sequencing of the MGMT promoter in DNA from a glioblastoma sample of a subject after the DNA has been treated with a reagent that converts an unmethylated cytosine to a uracil.

11. The use of claim 10, wherein the system or kit comprises primers for amplifying a DNA sequence of the MGMT promoter comprising SEQ ID NO: 1.

12. The use of claim 10, wherein the system or kit comprises primers for amplifying and sequencing a DNA sequence of the MGMT promoter comprising CpG44 and CpG61 of SEQ ID NO:1.

## Patentansprüche

1. Verfahren, umfassend:
(a) Durchführen einer Tiefensequenzierung des O-6-Methylguanin-DNA-Methyltransferase-Gens (MGMT) Promotors in DNA aus einer Glioblastom-Probe eines Subjekts, nachdem die DNA mit einem Reagenz behandelt wurde, das ein unmethyliertes Cytosin in Uracil umwandelt; und
(b) Nachweisen von Methylierung des MGMT-Promotors in der DNA,
wobei die detektierte Methylierung vorhersagt, ob das Subjekt auf eine Behandlung mit einem Alkylierungsmittel und/oder einer Strahlentherapie (RT) ansprechen wird und
wobei das Nachweisen von Methylierung des MGMT-Promotors das Nachweisen von Methylierung des MGMT-Promotors an einem CpG an Position 44 von SEQ ID NO: 1 und/oder das Nachweisen von Methylierung des MGMT-Promotors an einem CpG an Position 61 von SEQ ID NO: 1 umfasst.

2. Verfahren nach Anspruch 1, wobei das Alkylierungsmittel ausgewählt ist aus der Gruppe bestehend aus Temozolomid, Carmustin, Lomustin, Bendamustin, Uramustin, Cisplatin, Carboplatin, Mechlorethamin, Streptozocin, Cyclophosphamid, Ifosfamid, Melphalan, Chlorambucil, Busulfan, Thiotepa, Dacarbazin, Procarbazin, Altretamin, und Mitozolomid.

3. Verfahren nach Anspruch 1, wobei das Alkylierungsmittel Temozolomid ist.

4. Alkylierungsmittel und/oder Strahlentherapie zur Verwendung bei der Behandlung von Glioblastom in einem Subjekt, wobei das Subjekt zur Behandlung auf der Grundlage eines Verfahrens nach einem der Ansprüche 1 bis 3 ausgewählt ist.

5. Alkylierungsmittel und/oder Strahlentherapie zur Verwendung nach Anspruch 4, wobei das Alkylierungsmittel ausgewählt ist aus der Gruppe bestehend aus Temozolomid, Carmustin, Lomustin, Bendamustin, Uramustin, Cisplatin, Carboplatin, Mechlorethamin, Streptozocin, Cyclophosphamid, Ifosfamid, Melphalan, Chlorambucil, Busulfan, Thiotepa, Dacarbazin, Procarbazin, Altretamin und Mitozolomid.

6. Alkylierungsmittel und/oder Strahlentherapie zur Verwendung nach Anspruch 4, wobei das Alkylierungsmittel Temozolomid ist.

7. Verfahren nach einem der Ansprüche 1 bis 3, wobei vor der Durchführung der Tiefensequenzierung die behandelte DNA amplifiziert wird.

8. Verfahren nach einem der Ansprüche 1 bis 3, wobei vor der Durchführung der Tiefensequenzierung die behandelte DNA unter Verwendung von Prime amplifiziert wird, die nicht zwischen methylierter und nicht-methylierter DNA unterscheiden.

9. Verfahren nach einem der Ansprüche 1 bis 3, wobei die Durchführung der Tiefensequenzierung das Vervollständigen einer durchschnittlichen Probenabdeckung von wenigstens etwa 10³ Reads umfasst.

10. Verwendung eines Systems oder Kits zur Durchführung des Verfahrens nach einem der Ansprüche 1 bis 3, wobei das System oder Kit eine oder mehrere Komponenten zur Durchführung der Tiefensequenzierung des MGMT-Promotors in DNA aus einer Glioblastom-Probe eines Subjekts umfasst, nachdem die DNA mit einem Reagenz behandelt wurde, das ein unmethyliertes Cytosin in ein Uracil umwandelt.

11. Verwendung nach Anspruch 10, wobei das System oder Kit Primer zur Amplifikation einer DNA-Sequenz des MGMT-Promotors umfasst, die SEQ ID NO: 1 umfasst.

12. Verwendung nach Anspruch 10, wobei das System oder der Kit Primer zum Amplifizieren und Sequenzieren einer DNA-Sequenz des MGMT-Promotors umfasst, die CpG44 und CpG61 von SEQ ID NO: 1 umfasst.

## Revendications

1. Procédé comprenant les étapes consistant à :
(a) réaliser un séquençage profond du promoteur de gène de la O-6-méthylguanine-ADN méthyltransférase (MGMT) dans l'ADN provenant d'un échantillon de glioblastome d'un sujet après que l'ADN a été traité avec un réactif qui convertit une cytosine non méthylée en uracile ; et
(b) détecter la méthylation du promoteur de MGMT dans l'ADN,
dans lequel la méthylation détectée prédit si le sujet répondra au traitement avec un agent alkylant et/ou à une radiothérapie (RT) et
dans lequel la détection de la méthylation du promoteur de MGMT comprend la détection de la méthylation du promoteur de MGMT au niveau de CpG à la position 44 de la SEQ ID NO : 1 et/ou la détection de la méthylation du promoteur de MGMT au niveau de CpG à la position 61 de la SEQ ID NO : 1.

2. Procédé de la revendication 1, dans lequel l'agent alkylant est choisi dans le groupe consistant en le témozolomide, la carmustine, la lomustine, la bendamustine, l'uramustine, le cisplatine, le carboplatine, la méchloréthamine, la streptozocine, le cyclophosphamide, l'ifosfamide, le melphalan, le chlorambucil, le busulfan, le thiotépa, la dacarbazine, la procarbazine, l'altrétamine, et le mitozolomide.

3. Procédé de la revendication 1, dans lequel l'agent alkylant est le témozolomide.

4. Agent alkylant et/ou radiothérapie pour utilisation dans le traitement de glioblastome chez un sujet, où le sujet est sélectionné pour le traitement sur la base d'un procédé tel que revendiqué dans l'une quelconque des revendications 1 à 3.

5. Agent alkylant et/ou radiothérapie pour utilisation selon la revendication 4, dans lequel l'agent alkylant est choisi dans le groupe consistant en le témozolomide, la carmustine, la lomustine, la bendamustine, l'uramustine, le cisplatine, le carboplatine, la méchloréthamine, la streptozocine, le cyclophosphamide, l'ifosfamide, le melphalan, le chlorambucil, le busulfan, le thiotépa, la dacarbazine, la procarbazine, l'altrétamine, et le mitozolomide.

6. Agent alkylant et/ou radiothérapie pour utilisation selon la revendication 4, où l'agent alkylant est le témozolomide.

7. Procédé de l'une des revendications 1 à 3, dans lequel, avant de réaliser un séquençage profond, l'ADN traité est amplifié.

8. Procédé de l'une des revendications 1 à 3, dans lequel, avant de réaliser un séquençage profond, l'ADN traité est amplifié en utilisant des amorces qui ne distinguent pas entre l'ADN méthylé et non méthylé.

9. Procédé de l'une des revendications 1 à 3, dans lequel la réalisation d'un séquençage profond comprend l'accomplissement d'une couverture d'échantillon moyenne d'au moins environ 10³ lectures.

10. Utilisation d'un système ou d'un kit pour réaliser le procédé de l'une des revendications 1 à 3, le système ou kit comprenant un ou plusieurs composants pour réaliser un séquençage profond du promoteur de MGMT dans l'ADN provenant d'un échantillon de glioblastome d'un sujet après que l'ADN a été traité avec un réactif qui convertit une cytosine non méthylée en uracile.

11. Utilisation de la revendication 10, dans laquelle le système ou kit comprend des amorces pour amplifier une séquence d'ADN du promoteur de MGMT comprenant la SEQ ID NO : 1.

12. Utilisation de la revendication 10, dans laquelle le système ou kit comprend des amorces pour amplifier et séquencer une séquence d'ADN du promoteur de MGMT comprenant CpG44 et CpG61 de la SEQ ID NO : 1.
